# EUROPEAN PATENT APPLICATION

(11) **EP 4 434 576 A1**
(43) Date of publication of application: **25.09.2024**
(21) Application number: 24164260.2
(22) Date of filing: 18.03.2024
(51) Int. Cl.: A61N 2/02

(54) **CANCER TREATMENT APPARATUS**

(30) Priority: 23.03.2023 JP 2023046640
(71) Applicant: Ricoh Company, Ltd., Tokyo 143-8555 (JP); Public University Corporation Yokohama City University, Yokohama-shi, Kanagawa 236-0027 (JP)
(72) Inventor: Kishi, Kazuhito, Tokyo, 143-8555 (JP); Yamaguchi, Takashi, Tokyo, 143-8555 (JP); Hasegawa, Motokazu, Tokyo, 143-8555 (JP); Umemura, Masanari, Kanagawa, 236-0027 (JP); Ishikawa, Yoshihiro, Kanagawa, 236-0027 (JP)
(74) Representative: Marks & Clerk LLP

(57) **Abstract**

An alternating magnetic field having a frequency, at which a sufficient antitumor effect can be obtained, is enabled to be applied to a living body while restricting an amount of heat generation. A cancer treatment apparatus (100) includes a magnetic field generator (50) including a coil (30) configured to generate an alternating magnetic field to be applied to a living body (P), and a cooler (10) configured to cool a magnetic field application part of the living body (P) in which a temperature rises in response to application of the alternating magnetic field.

## Description

### BACKGROUND OF THE INVENTION

### Field of the Invention

The present disclosure relates to a cancer treatment apparatus.

### Description of the Related Art

Hyperthermia apparatuses including a ferromagnetic ferrite particle-containing ceramic heating element to be embedded near an in-vivo diseased site, and configured to put the diseased site within an alternating magnetic field to be generated from a coil, to thereby induce heat generation from the diseased site, and heat and treat the diseased site have been known (for example, see Japanese Unexamined Patent Application Publication No. 02-88059).

Cancer treatment apparatuses configured to apply an alternating magnetic field in a frequency range, in which a cancer cell proliferation inhibiting effect can be obtained, to a diseased tissue from a magnetic field generator that is deployed to surround a living body have been known (for example, see International Publication No. WO 2018/097185).

### SUMMARY OF THE INVENTION

According to an embodiment of the present disclosure, a cancer treatment apparatus includes a magnetic field generator including a coil configured to generate an alternating magnetic field to be applied to a living body, and a cooler configured to cool a magnetic field application part of the living body in which a temperature rises in response to application of the alternating magnetic field. The cancer treatment apparatus according to an embodiment of the present disclosure is not based on the hyperthermic effect like a hyperthermia apparatus, but utilizes the antitumor effect of a magnetic field itself as in International Publication No. WO 2018/097185.

### BRIEF DESCRIPTION OF THE DRAWINGS

FIG. 1 is a view illustrating an example of the configuration of a cancer treatment apparatus according to a first embodiment;
FIG. 2 is a front view illustrating an overview of a magnetic field generator of FIG. 1;
FIG. 3 is a top view illustrating an overview of the magnetic field generator of FIG. 1;
FIG. 4 is a cross-sectional view of the magnetic field generator along a line Z1-Z2 in FIG. 3;
FIG. 5 is a view illustrating an overview of a magnetic field generator of a cancer treatment apparatus according to a second embodiment;
FIG. 6 is a view illustrating an overview of a magnetic field generator of a cancer treatment apparatus according to a third embodiment;
FIG. 7 is a view illustrating an overview of a magnetic field generator of a cancer treatment apparatus according to a fourth embodiment;
FIG. 8 is a view illustrating an overview of a magnetic field generator of a cancer treatment apparatus according to a fifth embodiment;
FIG. 9 is a view illustrating an example of the configuration of a cancer treatment apparatus according to a sixth embodiment;
FIG. 10 is a cross-sectional view illustrating an overview of magnetic field generators of FIG. 9;
FIG. 11 is a cross-sectional oblique view illustrating an overview of the magnetic field generators of FIG. 9;
FIG. 12 is a view illustrating an example of the configuration of a cancer treatment apparatus according to a seventh embodiment;
FIG. 13 is a view illustrating an example in which an airflow is delivered to a patient by an air blower of FIG. 12;
FIG. 14 is a view illustrating an overview of a cancer treatment apparatus according to an eighth embodiment;
FIG. 15 is a view illustrating an example of the configuration of a cancer treatment apparatus according to a ninth embodiment;
FIG. 16 is a view illustrating an overview of a magnetic field generator of a cancer treatment apparatus according to a tenth embodiment;
FIG. 17 is a view illustrating another form of a cooling member in the tenth embodiment;
FIG. 18 is a drawing illustrating examples of waveforms of alternating magnetic fields applied to a patient by the cancer treatment apparatuses according to the embodiments mentioned above; and
FIG. 19 is a graph illustrating examples of temporal changes in the intensity of alternating magnetic fields applied to a patient by the cancer treatment apparatuses according to the embodiments mentioned above.

### DESCRIPTION OF THE EMBODIMENTS

For example, when an alternating magnetic field having a frequency of 100 kHz or higher is applied to a living body, heat generation occurs due to an induced current in the living body. When the body part of the living body, which is the target of magnetic field application, has a large size, a coil needs to have a large outer size (winding diameter). This causes a high induced current in the living body, and makes the amount of heat generation high. Hence, for example, when treating a diseased site that is present in the head or the abdomen using a coil situated around the head or the abdomen, it may be difficult to apply an alternating magnetic field that can provide a sufficient antitumor effect, to the diseased site for a predetermined period of time, due to constraints regarding the amount of heat generation.

In view of the above, an object of the present disclosure is to enable applying an alternating magnetic field having a frequency, at which a sufficient antitumor effect can be obtained, to a living body while restricting the amount of heat generation from the living body.

It is possible to enable applying an alternating magnetic field having a frequency, at which a sufficient antitumor effect can be obtained, to a living body while restricting the amount of heat generation from the living body.

Embodiments for carrying out the embodiments of the present disclosure will be described below in detail with reference to the drawings. The same components in the drawings will be denoted by the same reference numerals, and overlapping descriptions of such components will be omitted where appropriate. For example, the shapes of the components, the size proportions and the relative positional relationships between the components, and the like illustrated in the drawings, to which reference will be made in the following description, are mere examples, and the contents illustrated in the drawings are non-limiting. Moreover, there may be cases where, for example, the components and the like are exaggerated in order to make the description clear.

### <First embodiment>

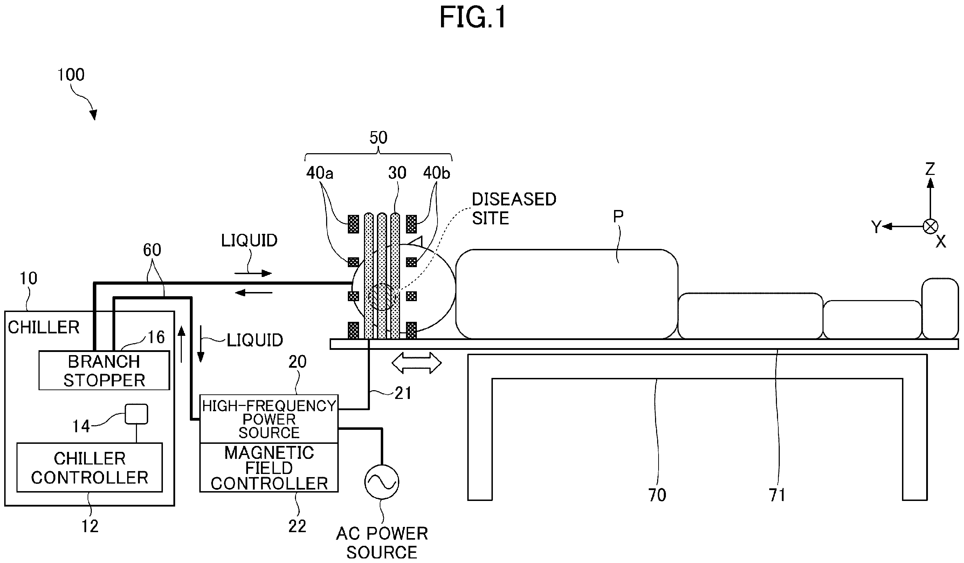

FIG. 1 is a view illustrating an example of the configuration of a cancer treatment apparatus according to a first embodiment. A cancer treatment apparatus 100 illustrated in FIG. 1 is a magnetic therapy apparatus configured to apply an alternating magnetic field to, for example, tumor cells, and expected to inhibit proliferation of cancer cells by this exact application of the alternating magnetic field. The cancer treatment apparatus 100 is different from, for example, a hyperthermia apparatus configured to apply a radio wave or the like and raise the temperature of tumor cells.

The cancer treatment apparatus 100 includes a chiller 10, a high-frequency power source 20, a magnetic field controller 22, and a magnetic field generator 50 including a coil 30, and cores 40a and cores 40b. The chiller 10, the high-frequency power source 20, and the magnetic field controller 22 are situated near a bed 70 on which a patient P lies. The patient P is an example of the living body. The chiller 10 and the coil 30 are examples of a cooler.

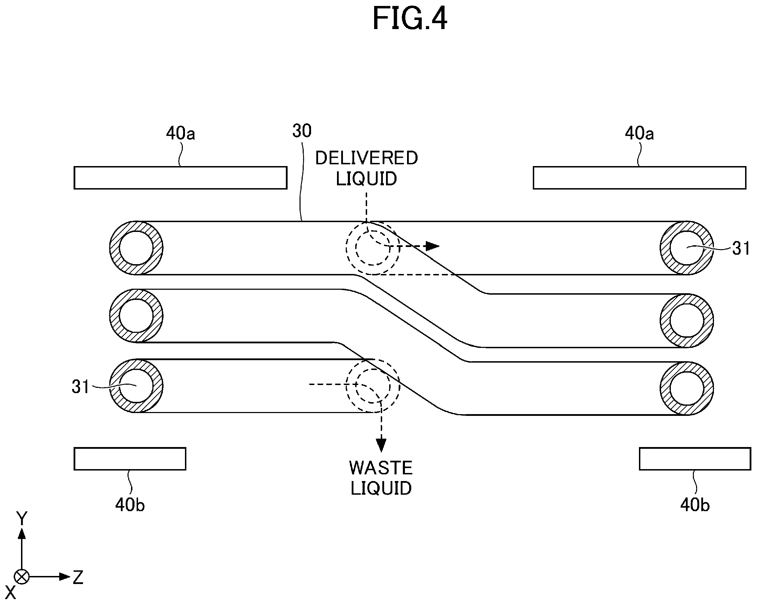

The chiller 10 is a cooling liquid circulating device, and includes a chiller controller 12, a temperature sensor 14, and a branch stopper 16. The temperature sensor 14 is an example of a temperature detector. The chiller 10 is configured to adjust the temperature of a cooling liquid to be supplied to the high-frequency power source 20 and the coil 30 to a previously set suitable temperature based on the temperature detected by the temperature sensor 14. The fluid circulated by the chiller 10 flows through a hollow portion 31 of the coil 30, as illustrated in FIG. 4. Hence, the temperature of the liquid detected by the temperature sensor 14 indicates the temperature of the coil 30.

In addition to the temperature sensor 14, a temperature sensor to be patched on the patient P, a temperature sensor to be situated near the coil 30, or a thermoviewer (thermography camera) or an infrared thermometer configured to measure the body temperature of the patient P may be provided. The chiller 10 may adjust the temperature of the liquid based not only on the temperature detected by the temperature sensor 14 but on the body temperature of the patient P or the temperature of the coil 30.

Moreover, the chiller 10 may also adjust the flow rate of the liquid to be circulated, while adjusting the temperature of the liquid. In a case where the temperature detected by the temperature sensor 14 becomes higher than a predetermined value, the chiller 10 may output an instruction to the high-frequency power source 20 to instruct the high-frequency power source 20 to reduce a current to apply. The branch stopper 16 is configured to output the liquid that is output from the chiller 10 into respective hoses 60 in a branched state. The hoses 60 are connected to the high-frequency power source 20 and the coil 30, respectively, in order to circulate the liquid between the chiller 10 and the coil 30, and between the chiller 10 and the high-frequency power source 20. The hoses 60 may be, for example, inflexible pipes or the like.

The temperature of the coil 30 is adjusted to, for example, a temperature lower than the temperature (e.g., from 42°C through 43°C) of tumor cells in a treatment using a hyperthermia apparatus, and is desirably adjusted to a temperature lower than the body temperature (e.g., from 35°C through 38°C) of the patient P to whom an alternating magnetic field is applied. It is more desirable to control the temperature of the coil 30 to be approximately room temperature (from 20°C through 30°C) of the room in which the cancer treatment apparatus 100 is situated. In order to inhibit dew condensation, the temperature of the coil 30 is preferably higher than or equal to the dew point in the apparatus environment of the cancer treatment apparatus 100 during application of an alternating magnetic field.

Effects applied to the patient P due to the coil 30 during application of an alternating magnetic field include: rising of the body temperature of the patient P in response to the heat of the coil 30, which has risen above room temperature due to an applied current, being transmitted to the patient P; and heat generation due to a current induced in the body of the patient P in response to the alternating magnetic field from the coil 30. By cooling the coil 30 to a temperature lower than the body temperature of the patient P by means of the circulating liquid, it is possible not only to inhibit the temperature in a region of the patient P to which the alternating magnetic field is applied from rising due to the heat of the coil 30, but to make the cooled coil 30 function as a cooling medium. By inhibiting rising of the temperature in the region of the patient P to which the alternating magnetic field is applied, it is possible to apply a higher current to the coil 30 and increase the intensity of the alternating magnetic field applied to the diseased site. This can improve the antitumor effect and shorten the treatment time. The region of the patient P to which the alternating magnetic field is applied is an example of the magnetic field application part.

Either or both of the flow rate and the temperature of the fluid may be adjusted based on the temperature that is sensed by the temperature sensor situated on the body surface of the patient P or on the coil 30, or by a thermoviewer. The treatment using the cancer treatment apparatus 100, in which an alternating magnetic field is applied to the patient P, is at a risk that the temperature of any metallic material embedded in the body such as a stent or the like may rise due to the alternating magnetic field. Hence, it is desirable to equip the cancer treatment apparatus 100 with a metal detector configured to detect any metallic material in the body.

The liquid supplied from the chiller 10 to the high-frequency power source 20 and the coil 30 is not limited to water, and may be oils and various types of aqueous solutions. A non-conductive liquid such as a fluorine-based organic compound and the like may be used in order to minimize trouble in the event of leakage of the liquid. When heat generation from the coil 30 is low, a gas such as air may be supplied to the coil 30.

The high-frequency power source 20 is configured to convert power supplied from an Alternating-Current (AC) power source such as a commercial power source to a high-frequency current of approximately from 100 kHz through 400 kHz in accordance with control of the magnetic field controller 22. The high-frequency current is applied to the coil 30 through a cable 21, to induce an alternating magnetic field from the coil 30. It has been experimentally confirmed that a high antitumor effect is obtained when the high-frequency current applied to the coil 30 is from 200 kHz through 250 kHz.

The magnetic field controller 22 is configured to control the intensity of an alternating magnetic field to be induced from the coil 30 by controlling the frequency, the amplitude, and the application time of the high-frequency current generated by the high-frequency power source 20. The frequency, the amplitude, and the application time of the high-frequency current, which the magnetic field controller 22 controls the high-frequency power source 20 to generate, are previously programmed before application of an alternating magnetic field to the patient P. The frequency, the amplitude, and the application time of the high-frequency current, which the magnetic field controller 22 controls the high-frequency power source 20 to generate, may be controlled such that the intensity of an alternating magnetic field applied to the patient P is fine-tuned in accordance with the degree of rising of the body temperature of the patient P.

Although not illustrated, a matching circuit may be situated between the high-frequency power source 20 and the coil 30, and a current from the high-frequency power source 20 may be applied to the coil 30 via the matching circuit. In this case, it is possible to generate a high current having a high frequency efficiently.

The bed 70 is equipped with a slider 71 configured to enable the patient P to be moved toward the magnetic field generator 50, and is situated near the magnetic field generator 50. The bed 70 is made of a non-magnetic, non-conductive material so as not to be affected by an alternating magnetic field generated by the magnetic field generator 50. By the slider 71 enabling the patient P to be moved toward the magnetic field generator 50, it is possible to adjust the relative positions of the diseased site of the patient P and the coil 30 and apply a suitable alternating magnetic field to the diseased site without stressing the patient P.

In the example illustrated in FIG. 1, the patient P has a diseased site (a tumor due to cancer cells) in the head. Hence, the coil 30 is formed in a size into which the head of the patient P moved together with the slider 71 can be inserted. That is, the inner diameter of the coil 30 is greater than the outer contour of the head of the patient P having the diseased site.

The magnetic field generator 50 may be situated to match the position of a diseased part in anywhere other than the head. For example, when an arm, a leg, or the like has a diseased site, it is possible to insert the diseased site into the space inside the coil 30 and treat the diseased site. The coil 30 may also be formed in a size (inner diameter) matching the shape of the diseased site to which an alternating magnetic field is applied. By forming the coil 30 of the magnetic field generator 50 in various shapes, it is possible to apply an alternating magnetic field having a predetermined intensity to also a diseased site present at a deep position in the body.

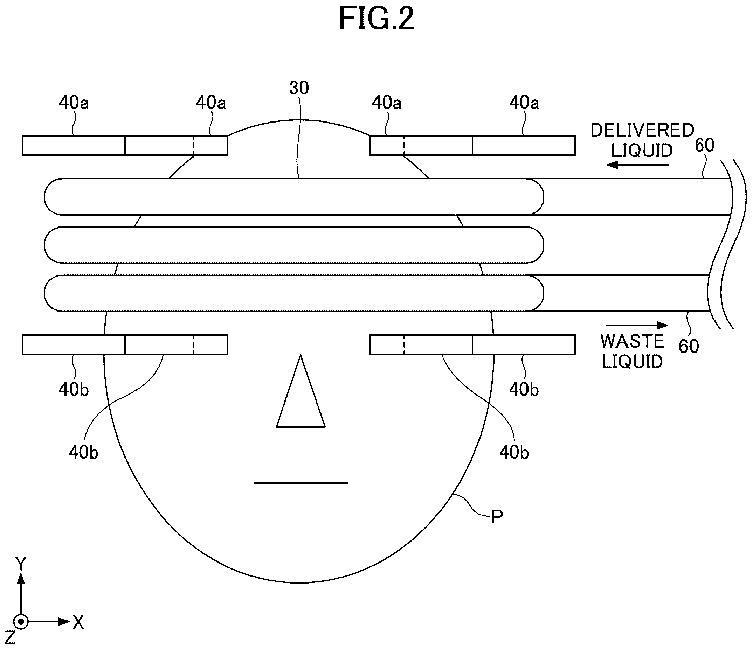

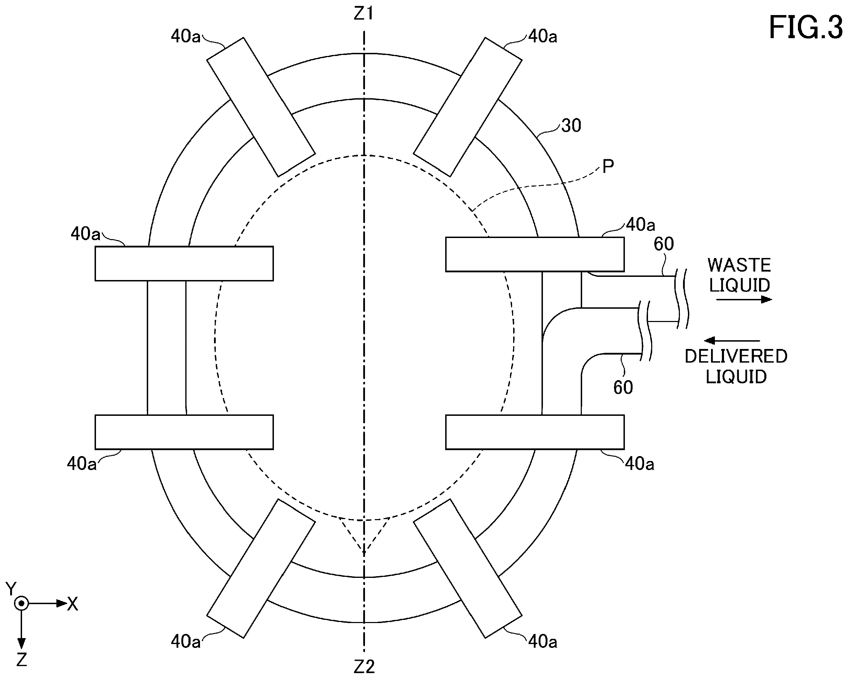

FIG. 2 is a front view illustrating an overview of the magnetic field generator 50 of FIG. 1. FIG. 3 is a top view illustrating an overview of the magnetic field generator 50 of FIG. 1. FIG. 4 is a cross-sectional view of the magnetic field generator 50 along a line Z1-Z2 in FIG. 3. The coil 30 is made of a pipe-shaped conductive material including a hollow portion 31 and having a circular transverse-sectional shape. In the present specification, a transverse section of the coil (or the conductive material of the coil) is defined as a cross-section orthogonal to the longer direction of the conductive material.

The liquid (delivered liquid) supplied from the chiller 10 through the hose 60 is supplied to the hollow portion 31 at one end of the coil 30. The liquid flowing through the hollow portion 31 of the coil 30 absorbs heat generated from the coil 30, and is returned to the chiller 10 through the hose 60 as a waste liquid.

The coil 30 is made of a highly conductive material that has a low loss at a high frequency, such as copper or the like. The coil 30 has a wound shape conforming to the shape of the magnetic field application part (in this example, the head) of the patient P. By forming the coil 30 in a wound shape, it is possible to make an area, over which the coil 30 and the patient P face each other, large, and to improve the efficiency with which the body part to which the alternating magnetic field is applied is cooled. As a result, it is possible to inhibit rising of the body temperature of the body part to which the alternating magnetic field is applied.

The number of turns in the coil 30 is 3, but the coil 30 may be formed in a number of turns of approximately from 2 through 5. The cores 40a and the cores 40b are situated on the respective sides of the coil 30 in the coil axial direction, in order to reduce the size of the apparatus while inhibiting any effects on other devices by concentrating the magnetic flux generated from the coil 30 and reducing leakage of the magnetic field to the surroundings of the magnetic field generator 50. By a high-frequency current being flowed through the coil 30, an alternating magnetic field is generated.

If too many cores 40a and cores 40b are situated on the path of the magnetic field, the inductance of the coil 30 becomes excessively high, which may make power source designing and selection difficult. Hence, it is desirable to situate the plurality of cores 40a by spacing them as illustrated in FIG. 3. It is also desirable to situate the plurality of cores 40b by spacing them.

The intensity of an alternating magnetic field applied to the cancer cells in the diseased site of the patient P is 2 mT or higher, and preferably 10 mT or higher. In order to obtain an antitumor effect, it is desirable to apply an alternating magnetic field of 10 mT or higher to the diseased site continuously for 30 minutes or longer every day or every few days. It has come to be experimentally made clear that the cancer cell proliferation inhibiting effect is higher as the intensity of the alternating magnetic field is higher. However, in a case of applying an intense alternating magnetic field to the diseased site from the coil 30 having an inner diameter greater than the outer contour of the body part including the diseased site, there is a risk that the temperature in the region of the patient P to which the alternating magnetic field is applied may exceed a normal range (e.g., 41°C or lower). It does not follow that increasing the intensity of the alternating magnetic field without limit is good, and it is desirable to restrict the intensity of the alternating magnetic field to be lower than 40 mT, and, if possible, 30 mT or lower.

However, as described above, by cooling the coil 30 by means of the circulating liquid, it is possible not only to inhibit the temperature in the region of the patient P to which the alternating magnetic field is applied from rising due to the heat of the coil 30, but to make the cooled coil 30 function as a cooling medium. That is, by positioning the coil 30, which is set to be at a lower temperature than the body temperature of the patient P, in proximity to the magnetic field application part of the patient P, it is possible to inhibit rising of the temperature of the magnetic field application part in response to application of the alternating magnetic field.

By inhibiting rising of the temperature in the region of the patient P to which the alternating magnetic field is applied, it is possible to apply a higher current to the coil 30 and increase the intensity of the alternating magnetic field applied to the diseased site. This can improve the antitumor effect and shorten the treatment time.

In the first embodiment, by cooling the coil 30 based on the temperature detected by the temperature sensor 14, it is possible to make the coil 30 function as a cooling medium and to inhibit rising of the temperature of the magnetic field application part of the patient P to which an alternating magnetic field is applied. Because it is possible to inhibit rising of the body temperature due to the effects of alternating magnetic field application, it is possible to apply a higher alternating magnetic field, and to improve the antitumor effect. That is, it is possible to apply an alternating magnetic field having a frequency, at which a sufficient antitumor effect can be obtained, to the living body while restricting the amount of heat generation from the living body.

By forming the coil 30 in a wound shape, it is possible to make an area, over which the coil 30 and the patient P face each other, large, and to improve the efficiency with which the body part to which an alternating magnetic field is applied is cooled. As a result, it is possible to inhibit rising of the body temperature of the body part to which the alternating magnetic field is applied. By supplying a fluid into the coil 30 having a pipe shape including the hollow portion 31, it is possible to efficiently cool the coil 30 to have no temperature unevenness.

### <Second embodiment>

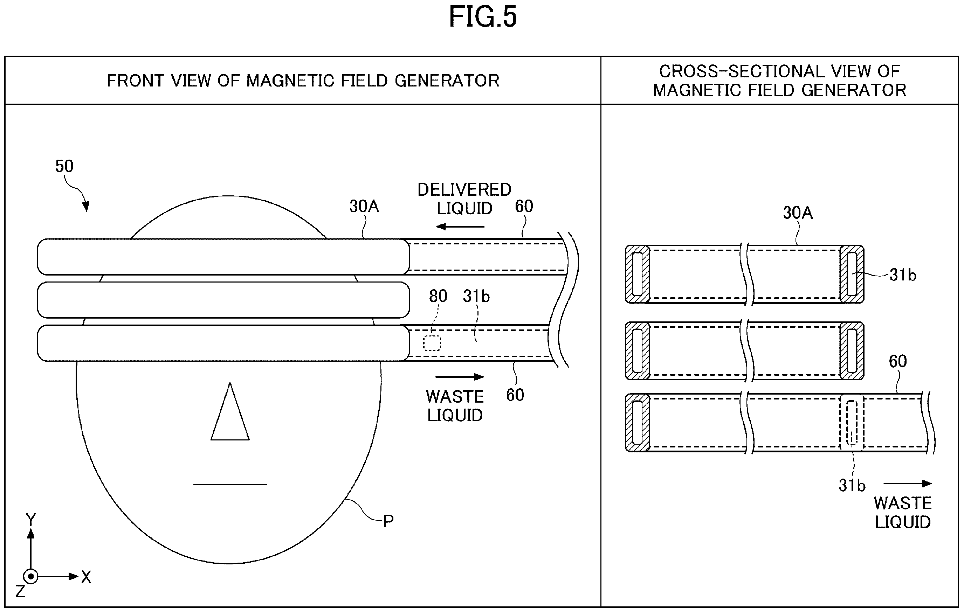

FIG. 5 is a view illustrating an overview of a magnetic field generator of a cancer treatment apparatus according to a second embodiment. Any configurational particulars that are the same as or similar to those in the first embodiment will be denoted by the same reference numerals, and detailed descriptions of such will be omitted.

The cancer treatment apparatus including the magnetic field generator 50 illustrated in FIG. 5 has the same configuration and functions as those of the cancer treatment apparatus 100 illustrated in FIG. 1, except that it includes a coil 30A instead of the coil 30 of FIG. 1 and includes a temperature sensor 80 instead of the temperature sensor 14 of FIG. 1. For example, the patient P has a diseased site (a tumor due to cancer cells) in the head. In FIG. 5, the cores 40a and the cores 40b illustrated in FIG. 2 are omitted. However, for example, the cores 40a and the cores 40b may be situated on the respective sides of the coil 30A in the coil axial direction.

The coil 30A is made of a conductive material such as copper or the like, which has a rectangular transverse-sectional shape having shorter sides and longer sides and includes a hollow portion 31b. The coil 30A has what is generally referred to as a flatwise-wound shape obtained by winding the conductive material in a state such that a surface of the conductive material having a dimension that is formed by a longer side of the rectangular transverse-sectional shape faces inward. Hence, it is possible to make a facing area, over which the coil 30A faces the body part of the patient P to which an alternating magnetic field is applied, larger than a facing area, over which a coil made of a material having a circular transverse-sectional shape faces that body part, and to improve the efficiency with which the body part to which the alternating magnetic field is applied is cooled. As a result, it is possible to inhibit rising of the body temperature of the body part to which the alternating magnetic field is applied, and to perform a more effective treatment.

For example, the temperature sensor 80 is attached to the internal surface of the hose 60 connected to a waste liquid outlet of the coil 30A, and configured to detect the temperature of a waste liquid discharged from the coil 30A. For example, the temperature sensor 80 is an optical probe sensor or the like that is unsusceptible to magnetic fields. The temperature sensor 80 may be attached to the waste liquid outlet of the coil 30A present in an outer circumferential portion of the coil 30A. Instead of the temperature sensor 80, an infrared thermometer, a thermoviewer, or the like that is configured to sense the temperature of the coil 30A or the hose 60 contactlessly may be provided.

By attaching the temperature sensor 80 on the waste liquid outlet side, it is possible to detect a temperature change in the body part of the patient P to which an alternating magnetic field is applied better than in a case where the temperature sensor 80 is attached on a delivered liquid inlet side. Moreover, it is possible to detect the temperature of the coil 30A more accurately than in a case of detecting the temperature of the liquid using the temperature sensor 14 situated in the chiller 10. For example, with the temperature sensor 14 of FIG. 1, there is a risk that the temperature of the liquid may change before the liquid returns to the chiller 10 from the coil 30A, and that the temperature of the coil 30A may be detected as being higher than the actual temperature.

The temperature sensor 80 may be patched on a part of the patient P facing the coil 30A. The temperature detected by the temperature sensor 80 indicates a relative temperature of the coil 30A, and indirectly indicates the temperature of the magnetic field application part cooled by the coil 30A.

The chiller 10 adjusts the temperature of the liquid supplied to the high-frequency power source 20 and the coil 30A to a previously set suitable temperature based on the temperature detected by the temperature sensor 80. Moreover, it is possible to make an alternating-current resistance lower when applying a high-frequency current to the coil 30A than when applying a high-frequency current to a coil having a circular transverse-sectional shape. Hence, it is possible to inhibit heat generation from the coil 30A, and to reduce power consumption by saving the cooling capability of the chiller 10.

Also in the second embodiment, it is possible to obtain the same effect as that in the first embodiment. For example, it is possible to inhibit rising of the temperature of the magnetic field application part of the patient P to which an alternating magnetic field is applied, by cooling the coil 30A based on the temperature detected by the temperature sensor 80 and making the coil 30A function as a cooling medium.

Moreover, in the second embodiment, it is possible to make a facing area, over which the coil 30A faces the body part of the patient P to which an alternating magnetic field is applied, larger than a facing area, over which a coil made of a material having a circular transverse-sectional shape faces that body part, and to improve the efficiency with which the body part to which the alternating magnetic field is applied is cooled. As a result, it is possible to effectively inhibit rising of the body temperature of the body part to which the alternating magnetic field is applied.

By controlling, for example, the flow rate and the like of the liquid based on the temperature detected by the temperature sensor 80 attached on the waste liquid outlet side of the coil 30A, it is possible to detect the temperature of the coil 30A more accurately, and to inhibit rising of the temperature of the magnetic field application part of the patient P suitably.

### <Third embodiment>

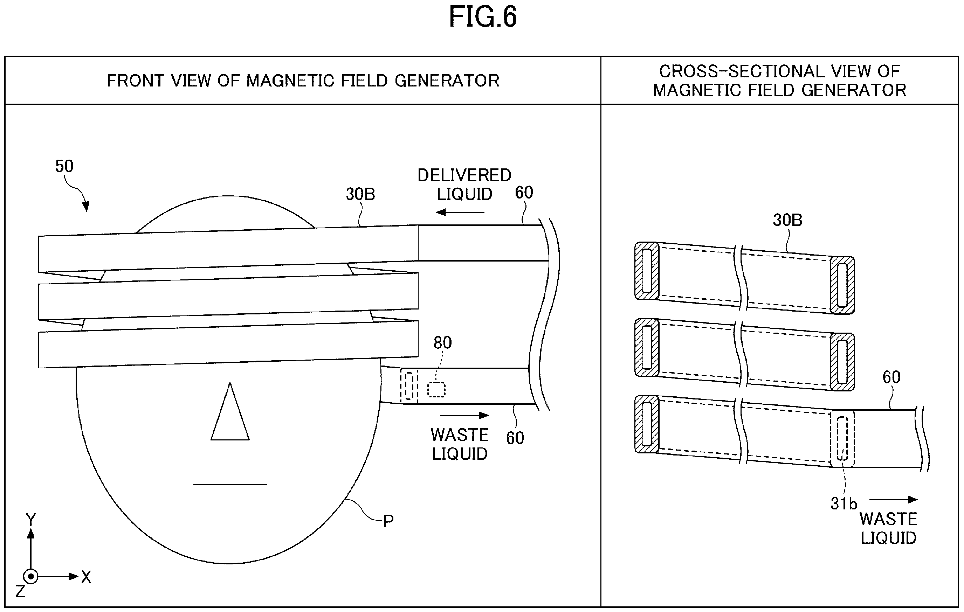

FIG. 6 is a view illustrating an overview of a magnetic field generator of a cancer treatment apparatus according to a third embodiment. Any configurational particulars that are the same as or similar to those in the first embodiment and the second embodiment will be denoted by the same reference numerals, and detailed descriptions of such will be omitted.

The cancer treatment apparatus including the magnetic field generator 50 illustrated in FIG. 6 has the same configuration and functions as those of the cancer treatment apparatus 100 illustrated in FIG. 1, except that it includes a coil 30B instead of the coil 30A of FIG. 5. For example, the patient P has a diseased site (a tumor due to cancer cells) in the head. In FIG. 6, the cores 40a and the cores 40b illustrated in FIG. 2 are omitted. However, for example, the cores 40a and the cores 40b may be situated on the respective sides of the coil 30B in the coil axial direction.

The coil 30B has the same shape as that of the coil 30A of FIG. 5, except that it is wound helically. That is, the coil 30B is made of a conductive material including a hollow portion 31b having a rectangular cross-sectional shape having shorter sides and longer sides. The coil 30B is wound in a state such that a surface of the conductive material having a dimension that is formed by a longer side of the rectangular cross-sectional shape faces inward. By winding the coil 30B helically, it is possible to avoid the coil being bent at some portions as illustrated in, for example, FIG. 4. Hence, it is possible to form an inexpensive coil 30B easily.

Also in the third embodiment, it is possible to obtain the same effect as that in the first embodiment and the second embodiment. In addition, in the third embodiment, by winding the coil 30B helically, it is possible to avoid the coil being bent at some portions as illustrated in, for example, FIG. 4. Hence, it is possible to form an inexpensive coil 30B easily.

### <Fourth embodiment>

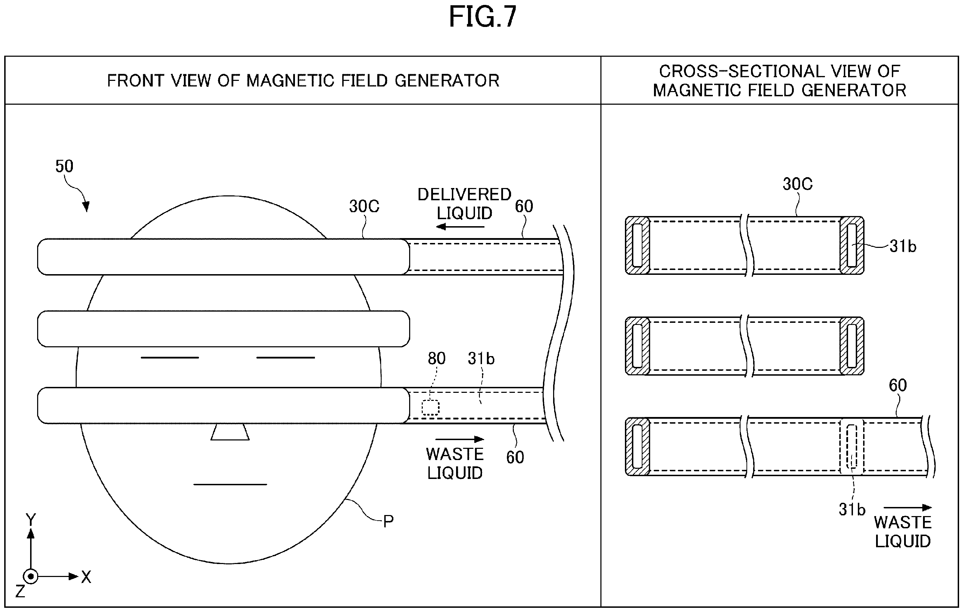

FIG. 7 is a view illustrating an overview of a magnetic field generator of a cancer treatment apparatus according to a fourth embodiment. Any configurational particulars that are the same as or similar to those in the first embodiment to the third embodiment will be denoted by the same reference numerals, and detailed descriptions of such will be omitted.

The cancer treatment apparatus including the magnetic field generator 50 illustrated in FIG. 7 has the same configuration and functions as those of the cancer treatment apparatus 100 illustrated in FIG. 1, except that it includes a coil 30C instead of the coil 30A of FIG. 5. For example, the patient P has a diseased site (a tumor due to cancer cells) in the head. In FIG. 7, the cores 40a and the cores 40b illustrated in FIG. 2 are omitted. However, for example, the cores 40a and the cores 40b may be situated on the respective sides of the coil 30C in the coil axial direction.

In the present embodiment, the interval between conductive portions in the coil 30C per winding is set to be greater than the interval between conductive portions in the coil 30A of FIG. 5 per winding. The other structural particulars of the magnetic field generator 50 are the same as those in the structure of the magnetic field generator 50 of FIG. 5. By making the interval between conductive portions in the coil 30C greater, it is possible to reduce an alternating-current resistance due to a near-field interaction when applying a high-frequency current to the coil 30C. Moreover, for example, it is possible to align the gap between conductive portions to come at the position of the eyes of the patient P. Hence, it is possible to not block the patient P's field of vision, and to reduce uneasiness that may be felt by the patient P during the treatment.

Also in the fourth embodiment, it is possible to obtain the same effect as that in the foregoing embodiments. In addition, in the fourth embodiment, by making the interval between conductive portions in the coil 30C greater, it is possible to reduce an alternating-current resistance due to a near-field interaction. Moreover, by making the interval between conductive portions in the coil 30C greater, it is possible to ensure the patient P an eyesight, and to reduce uneasiness that may be felt by the patient P during the treatment.

### <Fifth embodiment>

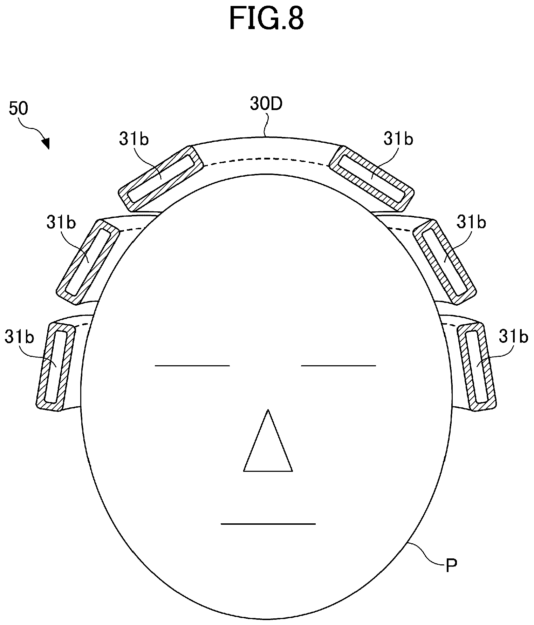

FIG. 8 is a view illustrating an overview of a magnetic field generator of a cancer treatment apparatus according to a fifth embodiment. Any configurational particulars that are the same as or similar to those in the first embodiment to the fourth embodiment will be denoted by the same reference numerals, and detailed descriptions of such will be omitted.

The cancer treatment apparatus including the magnetic field generator 50 illustrated in FIG. 8 has the same configuration and functions as those of the cancer treatment apparatus 100 illustrated in FIG. 1, except that it includes a coil 30D instead of the coil 30A of FIG. 5. For example, the patient P has a diseased site (a tumor due to cancer cells) in the head. In FIG. 8, the cores 40a and the cores 40b illustrated in FIG. 2 are omitted. However, for example, the cores 40a and the cores 40b may be situated along the external surface of the coil 30D.

The coil 30D has an internal surface having a hemispherical shape conforming to the shape of a head. Just the same as the coil 30A of FIG. 5, the coil 30D is made of a conductive material including a hollow portion 31b having a rectangular cross-sectional shape having shorter sides and longer sides, and the coil 30D is wound in a state such that a surface of the conductive material having a dimension that is formed by a longer side of the rectangular cross-sectional shape faces inward. Similarly to the case of FIG. 5, a temperature sensor 80 may be situated at a position in the hose 60 connected to the coil 30D, the position being close to the coil 30D, and the operation of the chiller 10 may be controlled based on the temperature detected by the temperature sensor 80.

By shaping the internal surface of the coil 30D, which includes the rectangular hollow portion 31b, in the hemispherical shape conforming to the shape of a head, it is possible to make the distance between: the surface of the coil 30D having the dimension formed by the longer side; and the surface of the head short irrespective of the positions of the conductive portions of the coil 30D. As a result, it is possible to further improve the efficiency with which the head to which an alternating magnetic field is applied is cooled, compared with the case of the coil 30A illustrated in FIG. 5. Note that by shaping the internal surface of the coil 30D in the hemispherical shape, it is possible to increase a breast cancer treatment effect.

Also in the fifth embodiment, it is possible to obtain the same effect as that in the foregoing embodiments. In addition, in the fifth embodiment, by shaping the internal surface of the coil 30D, which includes the rectangular hollow portion 31b, in the hemispherical shape conforming to the shape of a head, it is possible to make the distance between: the surface of the coil 30D having the dimension formed by the longer side; and the surface of the head short irrespective of the positions of the conductive portions of the coil 30D. As a result, it is possible to further improve the efficiency with which the head to which an alternating magnetic field is applied is cooled, compared with the case of the coil 30A illustrated in FIG. 5.

### <Sixth embodiment>

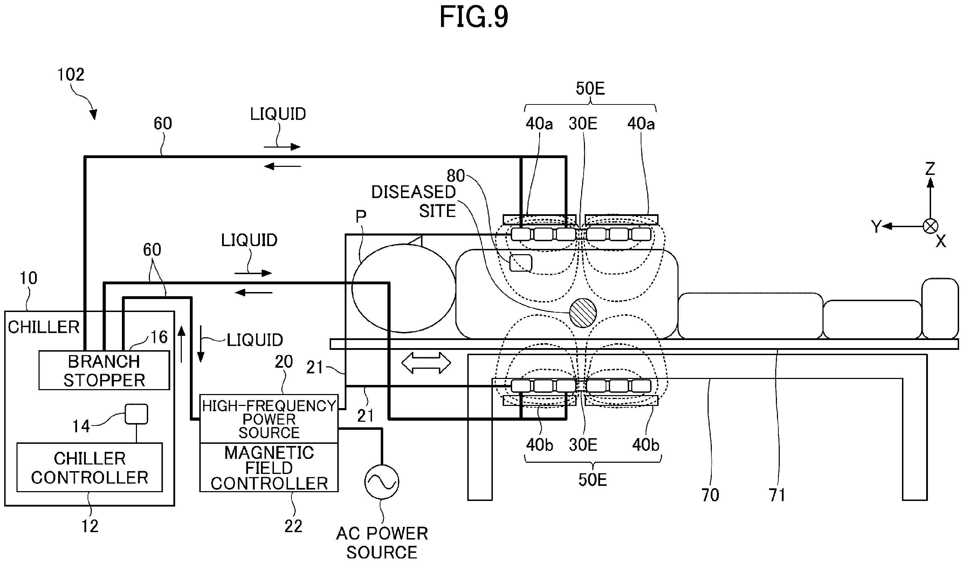

FIG. 9 is a view illustrating an example of the configuration of a cancer treatment apparatus according to a sixth embodiment. Any configurational particulars that are the same as or similar to those in the first embodiment will be denoted by the same reference numerals, and detailed descriptions of such will be omitted. The cancer treatment apparatus 102 illustrated in FIG. 9 includes a pair of magnetic field generators 50E facing each other via the patient P, and, for example, can apply an alternating magnetic field to a diseased site present in the abdomen of the patient P. Each magnetic field generator 50E includes a disk-shaped coil 30E, and cores 40a and cores 40b situated to face the coil 30E. By making the disk-shaped coils 30E face each other via the diseased site, it is possible to apply an intense alternating magnetic field to the diseased site also in a case where the diseased site is located at a deep position in the body.

The chiller 10 has the same configuration and function as those of the chiller 10 of FIG. 1, except that it is configured to circulate a liquid to the high-frequency power source 20 and to both of the pair of coils 30E via the branch stopper 16 in order to supply a liquid having a suitable temperature to the high-frequency power source 20 and to the pair of coils 30E. The high-frequency power source 20 is connected to both of the pair of coils 30E through cables 21, and is configured to output an alternating current to both of the pair of coils 30E in accordance with control of the magnetic field controller 22. The pair of coils 30E are configured to generate alternating magnetic fields in accordance with an alternating current. The functions of the bed 70 and the slider 71 are the same as those of the bed 70 and the slider 71 of FIG. 1. In the present embodiment, the patient P lying on the bed 70 is moved together with the slider 71 to the position at which the abdomen is sandwiched between the pair of magnetic field generators 50E.

In the present embodiment, for example, a temperature sensor 80 is patched on the magnetic field application part of the patient P. That is, the temperature sensor 80 is patched on a part of the patient P facing the coils 30E. By pasting the temperature sensor 80 on the patient P, it is possible to accurately detect the body temperature of the patient P.

The chiller 10 adjusts the temperature of the liquid supplied to the coils 30E to a previously set suitable temperature based on the temperature detected by the temperature sensor 80. The chiller 10 adjusts the temperature of the liquid supplied to the high-frequency power source 20 to a previously set suitable temperature based on the temperature detected by the temperature sensor 14. In a case where the temperature of the liquid to the coils 30E, the temperature being set based on the temperature detected by the temperature sensor 80, and the temperature of the liquid to the high-frequency power source 20, the temperature being set based on the temperature detected by the temperature sensor 14 are different from each other, the chiller 10 adjusts the temperature of the liquid to the lower temperature.

The chiller 10 may adjust the temperature of the liquid supplied to the coils 30E and to the high-frequency power source 20 to previously set respective suitable temperatures based on the temperatures detected by the temperature sensors 80 and 14. In this case, the chiller 10 includes a branch stopper 16 for the coils 30E and a branch stopper 16 for the high-frequency power source 20, and performs mutually independent temperature controls.

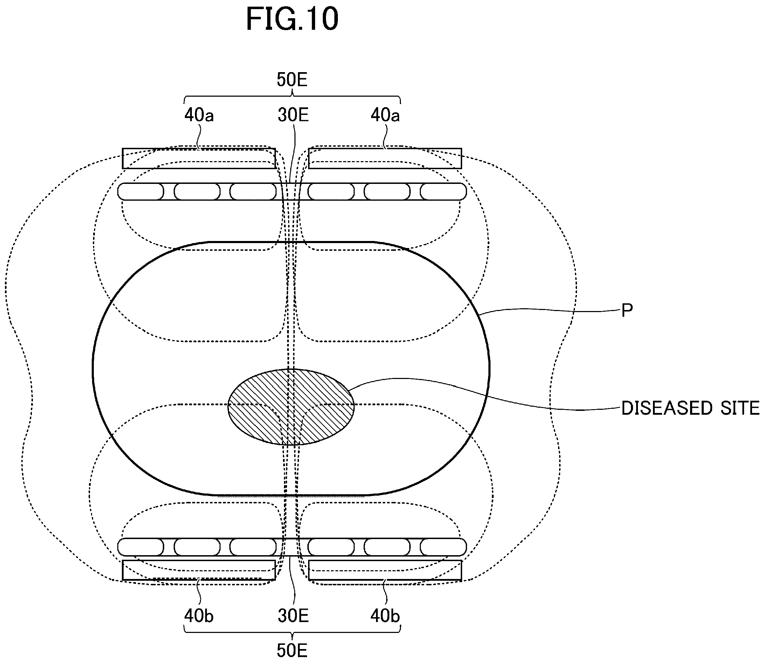

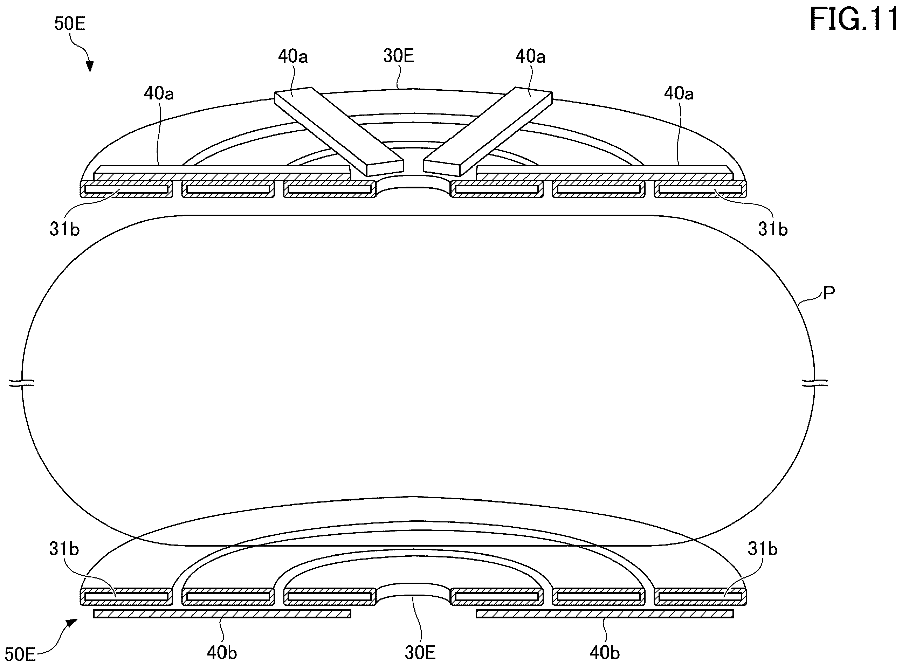

FIG. 10 is a cross-sectional view illustrating an overview of the magnetic field generators 50E of FIG. 9. FIG. 11 is a cross-sectional oblique view illustrating an overview of the magnetic field generators 50E of FIG. 9. The broken-line curves illustrated in FIG. 10 indicate the distribution of the intensity of the alternating magnetic fields generated from the coils 30E. Each coil 30E has what is generally referred to as a spiral shape, and is made of a conductive material that has a rectangular transverse-sectional shape having shorter sides and longer sides and includes a rectangular hollow portion 31b. The pair of coils 30E face each other by their surfaces that have a dimension formed by a longer side of the rectangular transverse-sectional shape. Although not illustrated, for example, liquid inlet portions are situated at portions of the circumferences of the spiral-shaped coils 30E, and liquid outlet portions are situated at the centers of the spiral-shaped coils 30E.

Hence, it is possible to make the facing area, over which the pair of coils 30E each face the body part of the patient P to which the alternating magnetic fields are applied, larger than the facing area, over which a coil made of a material having a circular transverse-sectional shape faces the body part, and to improve the efficiency with which the body part to which the alternating magnetic fields are applied is cooled. As a result, it is possible to inhibit rising of the body temperature of the body part to which the alternating magnetic fields are applied.

The cores 40a and the cores 40b each have a shape of a flat rectangular-parallelepiped, and are situated over surfaces of the pair of coils 30E opposite to the coils' facing surfaces and having a dimension formed by a longer side of the coils' rectangular transverse-sectional shape, in a state in which the cores 40a are spaced apart in the circumferential direction of the disk-shaped coil 30E and the cores 40b are spaced apart in the circumferential direction of the disk-shaped coil 30E. By situating the cores 40a and the cores 40b in proximity to the coils 30E, it is possible to intensify the alternating magnetic fields to be applied to the diseased site by inhibiting extra leakage of the alternating magnetic fields to the surroundings as illustrated in FIG. 10. Although illustration is omitted, it is possible to situate side cores configured to magnetically connect the cores 40a and the cores 40b that are situated over the side surfaces of the body, such that a closed magnetic circuit is formed to further reduce leaking magnetic fields and apply even more intense alternating magnetic fields to the living body.

Also in the sixth embodiment, it is possible to obtain the same effect as that in the foregoing embodiments. In addition, in the sixth embodiment, the pair of coils 30E each having a flat disk shape and each including the rectangular hollow portion 31b are situated such that their surfaces having the dimension formed by the longer side of the coils' rectangular transverse-sectional shape face each other. Hence, it is possible to apply alternating magnetic fields to the diseased site while inhibiting rising of the body temperature of the patient P positioned between the pair of coils 30E. Moreover, it is possible to apply an intense alternating magnetic field to the diseased site even in a case where the diseased site is located at a deep position in the body, because alternating magnetic fields can be applied from both sides of the patient P.

By pasting the temperature sensor 80 on the patient P, it is possible to accurately detect the body temperature of the patient P, and to make the chiller 10 perform liquid temperature control to follow the body temperature changes.

### <Seventh embodiment>

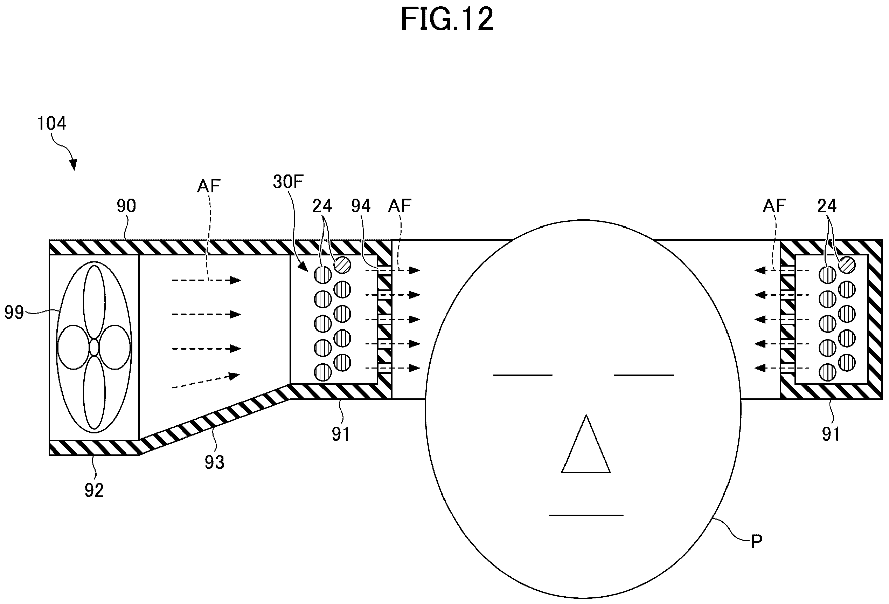

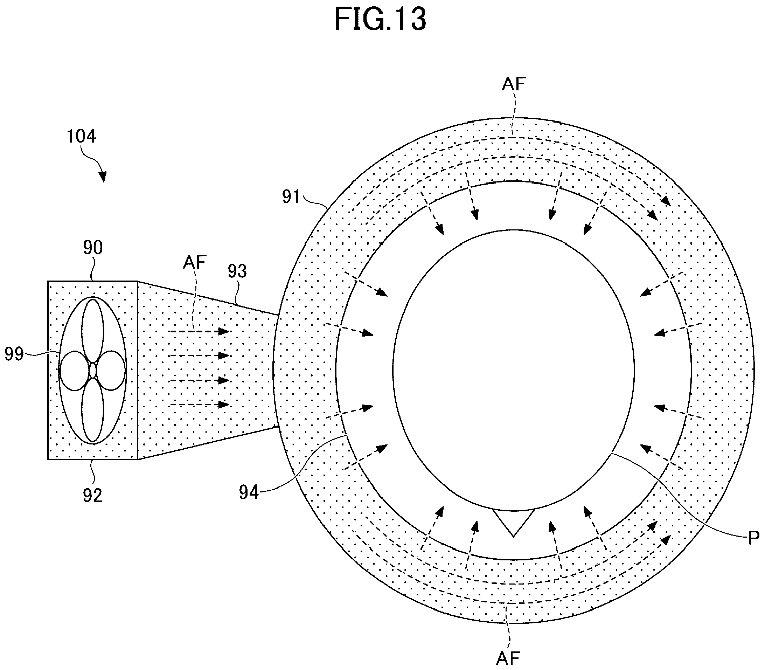

FIG. 12 and FIG. 13 are views illustrating overviews of a cancer treatment apparatus according to a seventh embodiment. The functions of the cancer treatment apparatus 104 illustrated in FIG. 12 are the same as the functions of the cancer treatment apparatus 100 illustrated in FIG. 1. For example, the alternating magnetic field generated by the cancer treatment apparatus 104 is the same as the alternating magnetic field generated by the cancer treatment apparatus 100 illustrated in FIG. 1.

The cancer treatment apparatus 104 includes a coil 30F formed by winding a non-hollow conductive material 24 such as a litz wire or the like, and an air blower 90 in which the coil 30F is stored. The air blower 90 includes a storage 91 for the coil 30F, an air blowing part 92 in which a fan 99 is situated, and an air passage 93 joining the storage 91 and the air blowing part 92 with each other.

The storage 91 has an annular transverse-sectional shape conforming to the shape of the coil 30F, and includes a plurality of air passage holes 94 in a cylinder-shaped inner wall of the storage 91. An airflow AF of air delivered into the storage 91 from the fan 99 through the air passage 93 cools the conductive material 24 by passing through gaps in the conductive material 24, and is evacuated through the air passage holes 94. The amount of heat generation from the coil 30F made of a litz wire is lower than the amount of heat generation from the hollow coil 30 illustrated in FIG. 1. Hence, should the airflow AF absorb the heat of the coil 30F, adjusting the airflow AF inhibits hot air from being emitted through the air passage holes 94.

For example, the head of the patient P in which a diseased site is present is positioned inside the cylinder-shaped inner wall of the storage 91, and receives an alternating magnetic field generated from the coil 30F. The airflow AF evacuated through the air passage holes 94 touches the head and inhibits rising of the temperature of the head. Air cooled by a heat pump or the like may be delivered to the storage 91 from the air blowing part 92. By having the coil 30F stored in the storage 91 and delivering air to the patient P through the storage 91, the cancer treatment apparatus 104 illustrated in FIG. 12 can cool the coil 30F utilizing the airflow AF for inhibiting rising of the temperature of the patient P.

Also in the seventh embodiment, it is possible to obtain the same effect as that in the foregoing embodiments. In addition, in the seventh embodiment, by storing the coil 30F in the storage 91 and delivering air to the patient P through the storage 91, it is possible to cool the coil 30F utilizing the airflow AF for inhibiting rising of the temperature of the patient P.

### <Eighth embodiment>

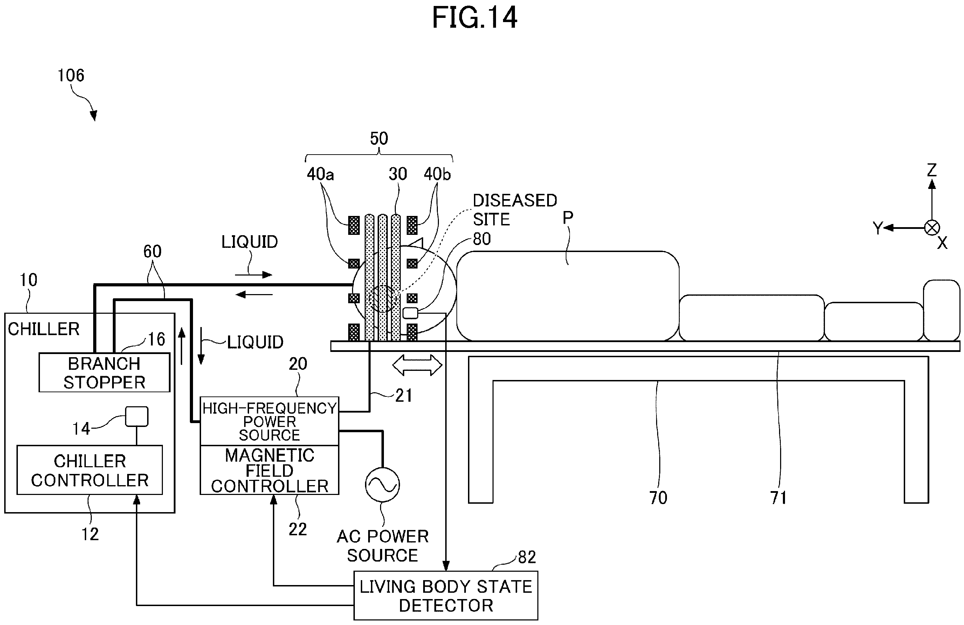

FIG. 14 is a view illustrating an example of the configuration of a cancer treatment apparatus according to an eighth embodiment. Any configurational particulars that are the same as or similar to those in the first embodiment will be denoted by the same reference numerals, and detailed descriptions of such will be omitted. A cancer treatment apparatus 106 illustrated in FIG. 14 includes a living body state detector 82 configured to acquire the body temperature measured by a temperature sensor 80 patched on the patient P, and control the chiller controller 12 and the magnetic field controller 22 based on the body temperature acquired. The configurational particulars and functions except the temperature sensor 80 and the living body state detector 82 are the same as the configurational particulars and functions of the cancer treatment apparatus 100 illustrated in FIG. 1.

The living body state detector 82 outputs temperature information indicating the body temperature acquired from the temperature sensor 80 to the chiller 10. The living body state detector 82 outputs an instruction to instruct stopping generating an alternating magnetic field to the magnetic field controller 22 in a case where the body temperature acquired from the temperature sensor 80 is higher than or equal to a previously set upper limit. Also in each of the foregoing embodiments in which the body temperature of the patient P is detected by the temperature sensor 80, the magnetic field controller 22 may be instructed to stop generating an alternating magnetic field in a case where the body temperature is higher than or equal to a previously set upper limit. Hence, it is possible to perform safe cancer treatment.

The chiller 10 adjusts the temperature of the liquid supplied to the coil 30 to a previously set suitable temperature based on the temperature detected by the temperature sensor 80. By pasting the temperature sensor 80 on the patient P, it is possible to accurately detect the body temperature of the patient P, and to make the chiller 10 perform liquid temperature control to follow the body temperature changes.

Also in the eighth embodiment, it is possible to obtain the same effect as that in the foregoing embodiments. In addition, in the eighth embodiment, it is possible to perform a safe cancer treatment by instructing the magnetic field controller 22 to stop generating an alternating magnetic field in a case where the body temperature acquired from the temperature sensor 80 is higher than or equal to a previously set upper limit.

### <Ninth embodiment>

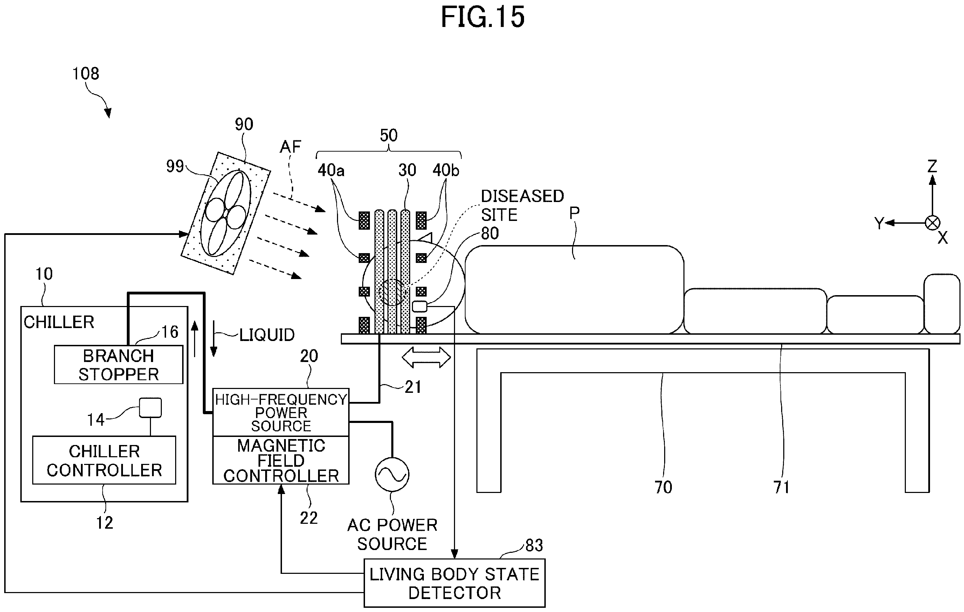

FIG. 15 is a view illustrating an example of the configuration of a cancer treatment apparatus according to a ninth embodiment. Any configurational particulars that are the same as or similar to those in the first embodiment and the eighth embodiment will be denoted by the same reference numerals, and detailed descriptions of such will be omitted. The cancer treatment apparatus 108 illustrated in FIG. 15 is the cancer treatment apparatus 106 illustrated in FIG. 14 to which the same air blower 90 as in FIG. 13 is added. The cancer treatment apparatus 108 includes a living body state detector 83 instead of the living body state detector 82 of FIG. 14.

The chiller 10 in the present embodiment is not connected to the coil 30, and does not have the function to cool the coil 30. The chiller 10 is configured to cool only the high-frequency power source 20. The air blower 90 is configured to cool the magnetic field application part and the coil 30.

The air blower 90 is situated at a position to direct the airflow AF to the magnetic field application part of the patient P. The rotation rate of the fan 99 of the air blower 90 is controlled by the living body state detector 83. The configurational particulars and functions except the air blower 90 and the living body state detector 83 are the same as the configurational particulars and functions of the cancer treatment apparatus 106 illustrated in FIG. 14.

The living body state detector 83 is configured to control the rotation rate of the fan 99 of the air blower 90 based on temperature information indicating the body temperature acquired from the temperature sensor 80, and adjust the amount of the airflow AF delivered to the magnetic field application part of the patient P. For example, the living body state detector 83 performs control to make the rotation rate of the fan 99 higher as the body temperature of the patient P is higher. The living body state detector 83 also outputs an instruction to instruct stopping generating an alternating magnetic field to the magnetic field controller 22 in a case where the body temperature acquired from the temperature sensor 80 is higher than or equal to a previously set upper limit, as does the living body state detector 82 of FIG. 14.

The air blower 90 may deliver air cooled by a heat pump or the like to the magnetic field application part of the patient P as the airflow AF. A wet cloth or the like may be attached to the magnetic field application part to be touched by the airflow AF from the air blower 90. By pasting a wet cloth or the like on the magnetic field application part, it is possible to improve the magnetic field application part cooling efficiency.

Also in the ninth embodiment, it is possible to obtain the same effect as that in the foregoing embodiments. In addition, in the ninth embodiment, by controlling the rotation rate of the fan 99 of the air blower 90 based on the temperature information indicating the body temperature acquired from the temperature sensor 80, it is possible to inhibit rising of the temperature of the magnetic field application part of the patient P to which the alternating magnetic field is applied.

### <Tenth embodiment>

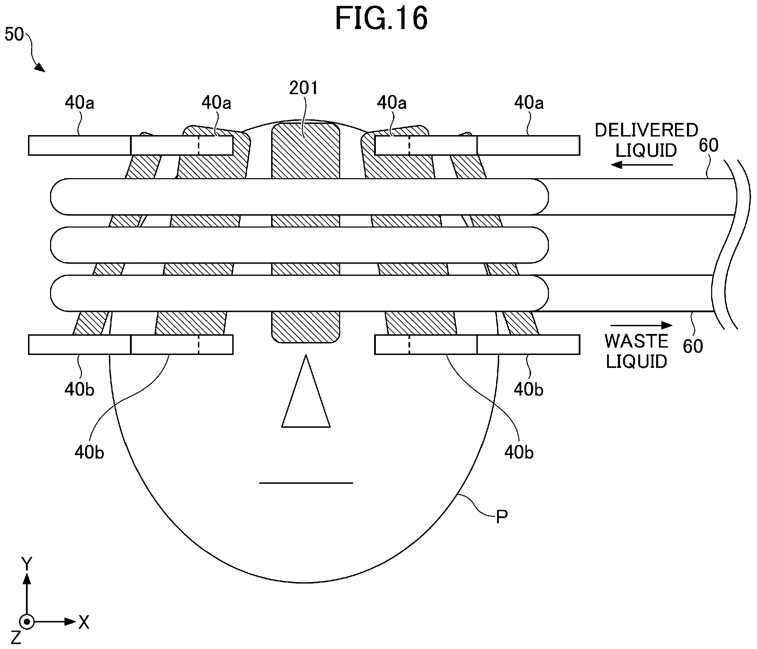

FIG. 16 is a view illustrating an overview of a magnetic field generator of a cancer treatment apparatus according to a tenth embodiment. Any configurational particulars that are the same as or similar to those in the foregoing embodiments will be denoted by the same reference numerals, and detailed descriptions of such will be omitted.

The cancer treatment apparatus including the magnetic field generator 50 illustrated in FIG. 16 has the same configuration and functions as those of the cancer treatment apparatus 100 illustrated in FIG. 1, except that a plurality of cooling members 201 are situated between the coil 30 of FIG. 2 and the head of the patient P. For example, the patient P has a diseased site (a tumor due to cancer cells) in the head.

Each cooling member 201 has a shape conforming to the outer shape of the magnetic field application part desired to be cooled. FIG. 16 illustrates an embodiment in which the plurality of cooling members 201 are concatenated with each other using a non-illustrated flexible material or the like and wound around the magnetic field application part. For example, by freezing the cooling members 201 in a freezer, it is possible to cool the cooling members 201 to below the freezing point. The cooling members 201 may be previously solidified in a state of conforming to the outer shape of the magnetic field application part.

In the present embodiment, by situating the cooling members 201 to contact the head of the patient P, it is possible to cool the magnetic field application part, and to inhibit rising of the temperature of the magnetic field application part, which may occur in response to an alternating magnetic field. In FIG. 16, the plurality of cooling members 201 are distributed in a direction circling about the normal to an alternating magnetic field to be generated, such that heat generation from the magnetic field application part is less likely to occur in response to application of the alternating magnetic field. Hence, by inhibiting heat generation from the cooling members 201 themselves, it is possible to maintain the cooling effect long, and to avoid replacement of the cooling members 201 during the treatment and interruption of the treatment.

The cooling members 201 do not need to be brought into direct contact with the patient P, and the cooling members 201 may be put inside an ice bag or the like having elasticity, such that the magnetic field application part may be cooled by a liquid in the ice bag cooled by the cooling members 201. Because the ice bag deforms to conform to the shape of the magnetic field application part, it is possible to improve the cooling function. Moreover, in order to reduce contact thermal resistance between the cooling members 201 and the body surface of the patient P, it is desirable to place a moisture-absorbent or water-absorbent sheet containing a liquid such as water or the like, or a gel-like heat transfer member.

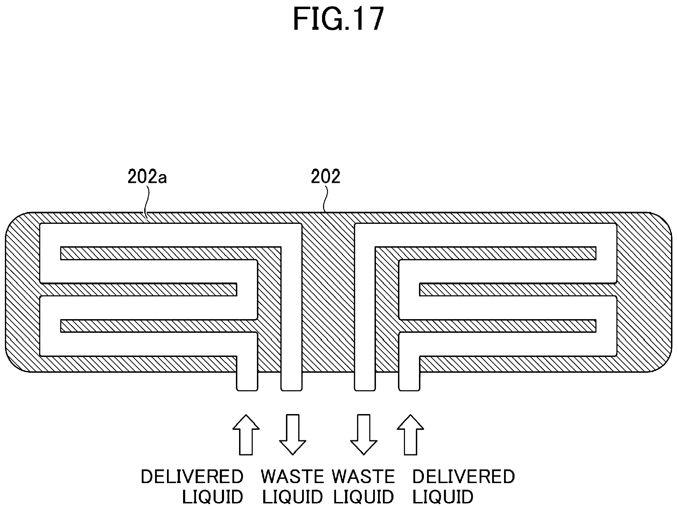

FIG. 17 is a view illustrating another form of a cooling member in the tenth embodiment. The cooling member 202 illustrated in FIG. 17 includes a hollow portion 202a through which a cooled liquid circulates. For example, the chiller 10 of FIG. 1 can circulate a cooled liquid through the cooling member 202. In the configuration illustrated in FIG. 17, even if the temperature of the cooling member 202 is relatively high, it is possible to maintain the cooling performance for a long time. Hence, it is possible to inhibit the patient P from being excessively cooled, and to continuously perform a treatment that takes a long time.

Also in the tenth embodiment, it is possible to obtain the same effect as that in the foregoing embodiments. In addition, in the tenth embodiment, by situating the cooling members 201 or the cooling member 202 along the magnetic field application part of the patient P, it is possible to cool the magnetic field application part, and to inhibit rising of the temperature of the magnetic field application part, which may occur in response to the magnetic field.

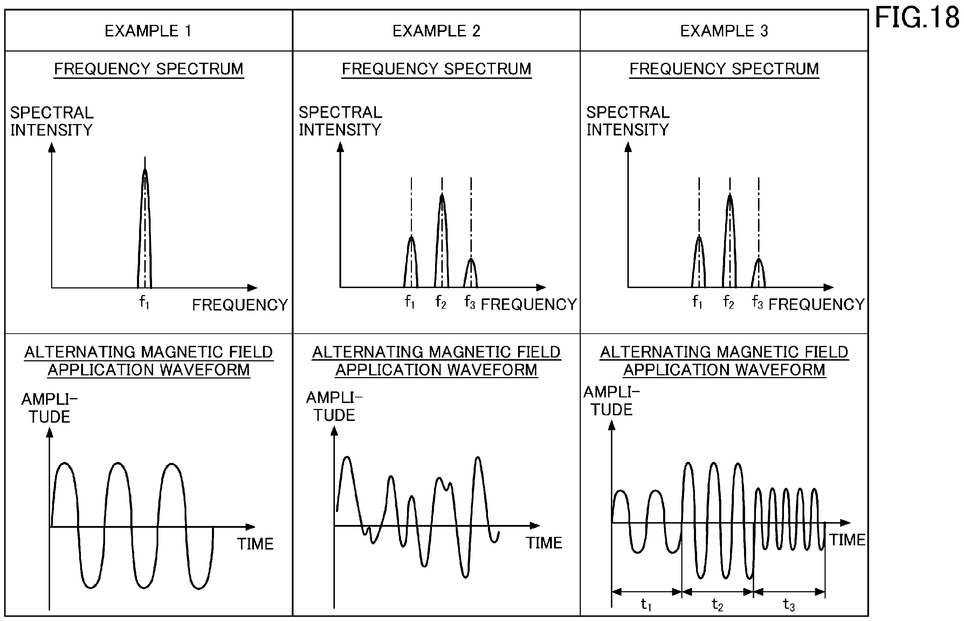

FIG. 18 is a drawing illustrating examples of waveforms of alternating magnetic fields applied to the patient by the cancer treatment apparatuses according to the foregoing embodiments. It is possible to appropriately change the waveforms of alternating magnetic fields to be applied to the diseased site of the patient in accordance with the types of the cancer cells to be treated.

For example, as illustrated in Example 1, the frequency spectrum of an alternating magnetic field may include a peak of one frequency f1. Alternatively, as illustrated in Example 2 and Example 3, the frequency spectrum of an alternating magnetic field may include peaks of a plurality of frequencies (in these examples, three frequencies f1, f2, and f3).

In Example 1, the alternating magnetic field has a waveform repeating a high level and a low level at a constant cycle. In Example 2, by superimposing the three frequencies f1, f2, and f3, it is possible to provide the alternating magnetic field with a complicated waveform in which unit waveform patterns of the frequencies f1, f2, and f3 are superimposed. In Example 3, by switching among the three frequencies f1, f2, and f3 once in every predetermined period t1, t2, or t3, it is possible to switch among the frequencies of alternating magnetic fields.

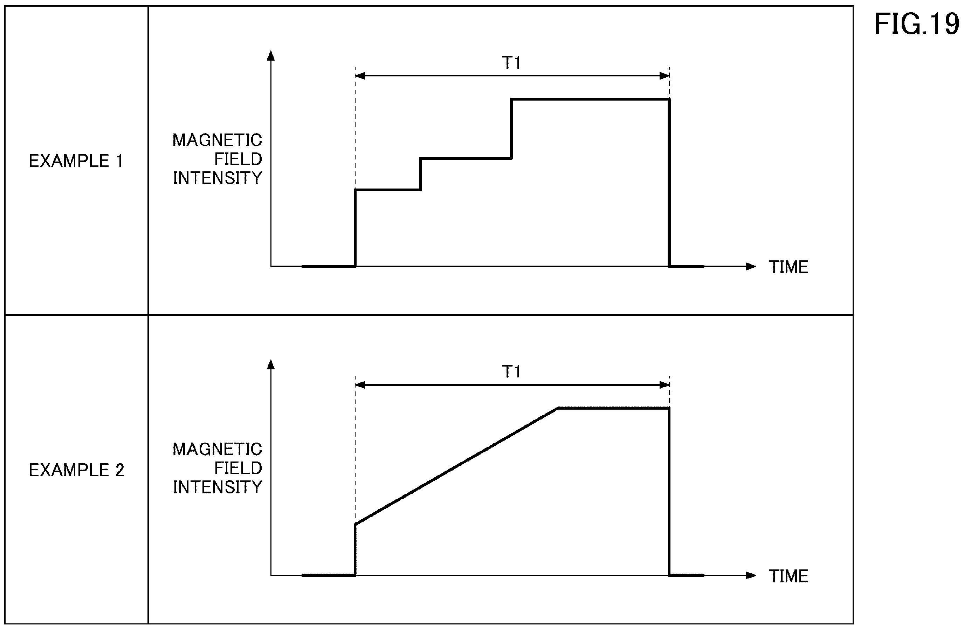

FIG. 19 is a graph illustrating examples of temporal changes in the intensity of alternating magnetic fields applied to a patient P by the cancer treatment apparatuses according to the foregoing embodiments. In a treatment of a diseased part, an alternating magnetic field is kept applied to the diseased part for a treatment time T1. Here, a maximum magnetic field is not kept applied for as long as the treatment time T1, but the intensity of the alternating magnetic field applied is adjusted to be lower in the initial period of the treatment time T1 than in the ending period of the treatment time T1. The intensity of the alternating magnetic field may be increased stepwise, and the maximum value may be maintained for a predetermined period of time as illustrated in Example 1. The intensity of the alternating magnetic field may be increased gradually, and the maximum value may be maintained for a predetermined period of time as illustrated in Example 2.

It has been found that applying an alternating magnetic field to a patient P raises the body temperature due to the effect of the alternating magnetic field. In the meantime, as the body temperature rises in response to application of the alternating magnetic field, the living body, attempting to reduce the body temperature, causes a reaction to promote heat dissipation by, for example, increasing the amount of blood circulation, increasing the amount of perspiration, and the like. Here, the amount of heat dissipation increases not instantly, but gradually over a period of time of approximately a few minutes through a few tens of minutes.

Hence, in a case of applying a maximum alternating magnetic field from the very beginning of alternating magnetic field application, at which the amount of heat dissipation is low, there is a risk that heat dissipation cannot catch up and the body temperature may sharply rise. Hence, as illustrated in FIG. 19, a relatively weak alternating magnetic field may be applied at the beginning of alternating magnetic field application, and the intensity of the alternating magnetic field applied may be gradually increased along with the progression of the heat dissipation promoting reaction in the living body. This makes it possible to apply a more intense alternating magnetic field while inhibiting rising of the body temperature. As a result, a better treatment effect can be obtained.

As described above, the antitumor effect is higher as the intensity of the alternating magnetic field applied to the patient P is higher. However, because the body temperature would rise due to heat generation due to an induced current, the intensity of the alternating magnetic field has an upper limit until which the alternating magnetic field can be applied to the patient P. It is said that if a human body exceeds 45°C even for a short period of time, the cells undergo an irreversible biochemical reaction and cannot be restored. Hence, it is necessary to set the intensity of the alternating magnetic field to be applied to the diseased site of the patient P, taking into consideration rising of the body temperature due to an induced current. For example, the upper limit of the body temperature that would rise in response to application of an alternating magnetic field is set to 41°C.

Aspects of the present disclosure are, for example, as follows.
<1> A cancer treatment apparatus, including:
   a magnetic field generator including a coil configured to generate an alternating magnetic field to be applied to a living body; and
   a cooler configured to cool a magnetic field application part of the living body in which a temperature rises in response to application of the alternating magnetic field.
<2> The cancer treatment apparatus according to <1>, further including:
   a temperature detector configured to detect a temperature of the coil or the temperature in the magnetic field application part,
   wherein the cooler cools the coil based on the temperature detected by the temperature detector, to cool the magnetic field application part via the coil thus cooled.
<3> The cancer treatment apparatus according to <2>,
   wherein the coil has a shape of a pipe including a hollow portion, and
   the cooler supplies a fluid for cooling the coil into the hollow portion, and controls either or both of a temperature and a flow rate of the fluid based on the temperature detected by the temperature detector.
<4> The cancer treatment apparatus according to <3>,
   wherein the coil has a rectangular transverse-sectional shape having longer sides and shorter sides, and the coil is shaped such that the living body is faced by one of two external surfaces of the coil, the two external surfaces being surfaces having a dimension that is formed by the longer sides.
<5> The cancer treatment apparatus according to <3> or <4>,
   wherein the coil has a wound shape conforming to a shape of the magnetic field application part.
<6> The cancer treatment apparatus according to any one of <3> to <5>,
   wherein the temperature detector detects the temperature of the fluid flowing through the coil, the temperature in the magnetic field application part, or the temperature of the coil.
<7> The cancer treatment apparatus according to <2>, further including:
   a cover member including a storage space in which the coil is stored, an inflow hole through which air is flowed into the storage space, and an outflow hole through which the air is flowed out of the storage space to the magnetic field application part,
   wherein the cooler controls an amount of the air flowed into the storage space based on the temperature detected by the temperature detector.
<8> The cancer treatment apparatus according to <2>,
   wherein the cooler includes a fan configured to generate an airflow toward the magnetic field application part, and controls a rotation rate of the fan based on the temperature detected by the temperature detector.

The present disclosure has been described based on the embodiments. However, the present disclosure is not limited to the requirements specified in the embodiments described above. These requirements can be modified within the range in which the purpose of the present disclosure is not missed, and can be suitably defined in accordance with the form of application.

## Claims

1. A cancer treatment apparatus, comprising:
a magnetic field generator including a coil configured to generate an alternating magnetic field to be applied to a living body; and
a cooler configured to cool a magnetic field application part of the living body in which a temperature rises in response to application of the alternating magnetic field.

2. The cancer treatment apparatus according to claim 1, further comprising:
a temperature detector configured to detect a temperature of the coil or the temperature in the magnetic field application part,
wherein the cooler is configured to cool the coil based on the temperature detected by the temperature detector, to cool the magnetic field application part via the coil thus cooled.

3. The cancer treatment apparatus according to claim 2, wherein the coil has a shape of a pipe including a hollow portion, and
the cooler is configured to supply a fluid for cooling the coil into the hollow portion, and to control either or both of a temperature and a flow rate of the fluid based on the temperature detected by the temperature detector.

4. The cancer treatment apparatus according to claim 3, wherein the coil has a rectangular transverse-sectional shape having longer sides and shorter sides, and the coil is shaped such that the living body is faced by one of two external surfaces of the coil, the two external surfaces being surfaces having a dimension that is formed by the longer sides.

5. The cancer treatment apparatus according to claim 3 or 4, wherein the coil has a wound shape conforming to a shape of the magnetic field application part.

6. The cancer treatment apparatus according to claim 3 or 4, wherein the temperature detector is configured to detect the temperature of the fluid flowing through the coil, the temperature in the magnetic field application part, or the temperature of the coil.

7. The cancer treatment apparatus according to any one of claims 2 to 6, further comprising:
a cover member including a storage space in which the coil is stored, an inflow hole through which air is flowed into the storage space, and an outflow hole through which the air is flowed out of the storage space to the magnetic field application part,
wherein the cooler controls an amount of the air flowed into the storage space based on the temperature detected by the temperature detector.

8. The cancer treatment apparatus according to any one of claims 2 to 7, wherein the cooler includes a fan configured to generate an airflow toward the magnetic field application part, and controls a rotation rate of the fan based on the temperature detected by the temperature detector.
